# EUROPEAN PATENT SPECIFICATION

(11) **EP 4 114 172 B1**
(45) Date of publication and mention of the grant of the patent: **06.05.2026**
(21) Application number: 21708223.9
(22) Date of filing: 26.02.2021
(51) Int. Cl.: A01K 67/0275, C12N 9/50, C12N 15/85

(54) **RODENT MODEL FOR HYPERKERATOSIS AND SEPSIS**
NAGETIERMODELL FÜR HYPERKERATOSE UND SEPSIS
MODÈLE DE RONGEUR POUR L'HYPERKÉRATOSE ET LA SEPTICÉMIE

(30) Priority: 02.03.2020 EP 20160475
(43) Date of publication of application: 11.01.2023
(73) Proprietor: Christian-Albrechts-Universität zu Kiel, 24118 Kiel (DE)
(72) Inventor: BECKER-PAULY, Christoph, 24229 Dänischenhagen (DE); PETERS, Florian, 8005 Zürich (CH)
(74) Representative: Taruttis, Stefan Georg
(86) International application number: PCT/EP2021/054936
(87) International publication number: WO 2021/175738

(56) References cited:
- JOHN E. BYLANDER ET AL: "Meprin Metalloprotease Deficiency Associated with Higher Mortality Rates and More Severe Diabetic Kidney Injury in Mice with STZ-Induced Type 1 Diabetes", JOURNAL OF DIABETES RESEARCH, vol. 2017, 1 January 2017 (2017-01-01), pages 1 - 11, XP055713452, ISSN: 2314-6745, DOI: 10.1155/2017/9035038
- HERZOG CHRISTIAN ET AL: "Role of meprin metalloproteinases in cytokine processing and inflammation", CYTOKINE, vol. 114, 30 December 2018 (2018-12-30), pages 18 - 25, XP085625039, ISSN: 1043-4666, DOI: 10.1016/J.CYTO.2018.11.032
- CHRISTOPH BECKER-PAULY ET AL: "The [alpha] and [beta] Subunits of the Metalloprotease Meprin Are Expressed in Separate Layers of Human Epidermis, Revealing Different Functions in Keratinocyte Proliferation and Differentiation", JOURNAL OF INVESTIGATIVE DERMATOLOGY, vol. 127, no. 5, May 2007 (2007-05-01), NL, pages 1115 - 1125, XP055713520, ISSN: 0022-202X, DOI: 10.1038/sj.jid.5700675
- CLAUDIA BRODER ET AL: "The metalloproteases meprin [alpha] and meprin &bgr;: unique enzymes in inflammation, neurodegeneration, cancer and fibrosis", BIOCHEMICAL JOURNAL, vol. 12, no. 2, March 2013 (2013-03-01), pages 390 - 264, XP055307276, ISSN: 0264-6021, DOI: 10.1042/BJ20121751
- LAI YU-CHING ET AL: "Intraarticular induction of interleukin-1 beta expression in the adult mouse, with resultant temporomandibular joint pathologic changes, dysfunction, and pain", ARTHRITIS & RHEUMATISM, WILEY INTERSCIENCE, US, vol. 54, no. 4, April 2006 (2006-04-01), pages 1184 - 1197, XP002476279, ISSN: 0004-3591, DOI: 10.1002/ART.21771
- HYEONHUI KIM ET AL: "Mouse Cre-LoxP system: general principles to determine tissue-specific roles of target genes", LABORATORY ANIMAL RESEARCH, vol. 34, no. 4, January 2018 (2018-01-01), pages 147, XP055713445, ISSN: 1738-6055, DOI: 10.5625/lar.2018.34.4.147

## Description

The present invention relates to a rodent model for hyperkeratosis and/or for sepsis and/or for systemic inflammation, which rodent has the advantage of developing hyperkeratosis and sepsis upon administration of an external stimulus at an age of the rodent that can be chosen freely. The rodent is e.g. for use in research, especially for use in processes for testing pharmaceutical compounds in respect of an effect on hyperkeratosis and/or on sepsis and/or on systemic inflammation. The animal is a rodent, e.g. a mouse, rat, rabbit, guinea pig. Generally herein, the non-human mammal is also simply referred to as animal. In the context of the invention, the animal is a rodent. The animal has the advantage that the sepsis and/or systemic inflammation is not elicited against an antigen, and hence the phenotype of sepsis and/or of systemic inflammation is not antigen-specific. Accordingly, the invention also relates to processes for testing pharmaceutical compounds, e.g. for use in screening test compounds, for their effect on hyperkeratosis and/or on sepsis and/or on systemic inflammation as induced in an animal model as provided herein.

The rodent of the invention is genetically manipulated to develop the phenotype of hyperkeratosis and sepsis upon activation by an external stimulus, which preferably is a synthetic compound. Accordingly, the invention also relates to a process for generating the animal model.

The rodent of the invention has the advantage of reproducibly generating a phenotype of hyperkeratosis and of sepsis without administration of toxins or other pro-inflammatory stimuli and without mechanically destroying epithelia or tissues of the rodent.

### State of the art

Stortz et al., ILAR Journal 90-105 (2017), review the advantages of using mice as animal models and describe murine sepsis models which are generated by exogenous administration of toxin or of a viable pathogen, or by puncture of intestines.

Holly et al., Kidney International 496-506 (2006), describe that upon induction of sepsis by puncture of intestines in rats, which resulted in acute kidney failure, changes of the concentration of a number of proteins in urine were found, the proteins including albumin, brush-border enzymes (e.g. meprin-1-alpha), and serine protease inhibitors. Meprin-1-alpha is mentioned as a potential biomarker and drug target.

Wang et al., SHOCK 141-147 (2011), describe that actinonin, a meprin-1-A inhibitor, should be evaluated as a therapeutic agent in targeting early and later organ-damaging effects of sepsis that was induced by puncturing the intestine subsequent to administration of actinonin. Herzog et al. in the review article Cytokine 18-25 (2019) quote that meprin α-KO mice upon sepsis induced by LPS showed reduction in pro-inflammatory cytokines IL-1β and TNF-α and exhibited less bladder edema, leukocyte infiltration and bladder permeability than wt mice. Herzog et al. conclude that meprin A contributes to renal and urogenital pathogenesis in LPS model of sepsis. Herzog et al. further quote that meprin α was identified as a susceptibility gene for inflammatory bowel disease in ulcerative colitis patients, and quote that meprin α-KO mice were more susceptible to DSS-induced experimental colitis and displayed more colon damage and inflammation than wt mice.

Herzog et al. only quote detrimental effects of knock-out of meprin α, and Herzog et al. do not relate to possible effects of inducing expression of meprin α.

Lai et al., Arthritis & Rheumatism 1184-1197 (2006) describe a mouse model that was genetically manipulated to allow conditional overexpression of IL-1β.

Becker-Pauly et al., Journal of Investigative Dermatology 1115-1125 (2007) in 5 patients diagnosed for Ichthyosis vulgaris, a disease model of retention hyperkeratosis, mentions high levels of meprin α. However, cultivated keratinocytes (HaCaT) were not influenced by meprin α. It is mentioned that meprin α may play a role in colorectal cancer and in melanoma formation or in wound healing.

Broder and Becker-Pauly, Biochem. Journal, 253-264 (2014) relates to meprin α-KO mice in relation to kidney damage and describes release of meprin α upon activation of TGF-α and EGF, leading to inflammatory effects, and describes an increase of melanoma metastasis. Meprin α is said to be overexpressed in skin fibroblasts of fibrotic tumours.

An effect of induced expression of meprin α in a genetically manipulated experimental animal cannot be inferred from the prior publications.

### Object of the invention

It is an object of the invention to provide an animal model, which generates hyperkeratosis and sepsis upon induction of an external stimulus, which stimulus does not require the administration of a toxin nor of a pro-inflammatory molecule to the animal, nor mechanically damaging the animal model, e.g. no puncturing or other damaging of intestines. Preferably, the animal model is genetically manipulated such that administration of an external stimulus, which is a chemical compound, results in a phenotype of hyperkeratosis and of sepsis.

### Description of the invention

The invention achieves the object by the features of the claims and especially by providing a rodent which is genetically manipulated to overexpress meprin α, at least or only in keratinocytes, under the control of an external stimulus. Meprin α can be overexpressed from an additional expression cassette that was introduced into the rodent by genetic manipulation. Accordingly, the rodent is genetically manipulated to contain a genetic element which in the presence of an external stimulus induces overexpression of meprin α from an expression cassette encoding meprin α. The meprin α can have the amino acid sequence of the endogenous meprin α, or the amino acid sequence of a heterologous meprin α.

The rodent can be genetically manipulated to contain an expression cassette encoding meprin α under the control of an inducible promoter, which promoter is inducible by an external stimulus and which promoter in the absence of the external stimulus does not express meprin α. Accordingly, upon presence of the external stimulus, the promoter is activated to express meprin α. Generally, the expression of meprin α under the control of the promoter that is inducible by an external stimulus is also termed overexpression of meprin α, because this expression is in addition to and is independent of any endogenous expression of meprin α. For introducing a promoter into a functional relationship to a meprin α encoding nucleic acid sequence, which encoding nucleic acid sequence can be a nucleic acid that is introduced along with the promoter, viral vectors can be used.

Further, the invention provides a process for generating the rodent as defined in the claims, which is genetically manipulated to contain an expression cassette encoding meprin α under the control of an inducible promoter, e.g. by genetically manipulating stem cells of the animal to contain an additional expression cassette for meprin α, and producing an animal from the genetically manipulated stem cells.

As the rodent develops hyperkeratosis and sepsis and/or systemic inflammation upon administration of the external stimulus that induces the expression of meprin α from the expression cassette introduced by genetic manipulation, the invention further provides a process for investigating the effect of compounds, e.g. for screening compounds in search of pharmaceutically active compounds, by providing the rodent of the invention, applying the external stimulus inducing the overexpression of meprin α from the expression cassette, and administering a compound prior to or subsequent to applying the external stimulus. This process makes use of the development of hyperkeratosis and/or sepsis and/or systemic inflammation in the rodent upon administration of an external stimulus, which rodent is genetically manipulated to comprise a promoter that is inducible by an external stimulus, and which promoter is functionally linked to a nucleic acid sequence encoding meprin α. As the overexpression of meprin α under the control of an inducible promoter by administration of the external stimulus that induces this promoter is sufficient for generating the phenotype of hyperkeratosis and sepsis and systemic inflammation, the process for generating this phenotype may consist of providing the genetically manipulated animal of the invention and administering the external stimulus, e.g. without administration of any additional toxin or other pro-inflammatory stimuli, and without mechanically destroying epithelia or tissues of the rodent.

Surprisingly, it was found that overexpressing meprin α in a rodent resulted in the generation of a phenotype, which resembles hyperkeratosis and sepsis and systemic inflammation. Herein, expression of meprin α that is in addition to normal expression of meprin α is also termed overexpression. This animal model, e.g. a mouse or rat, has shown the advantage of an apparently normal development, e.g. into an adult animal, and to develop hyperkeratosis and sepsis upon application of the external stimulus that induces the overexpression of meprin α, without administrating a toxin known to induce sepsis and without puncturing the intestine or otherwise surgically damaging the animal.

The additional expression cassette is under the control of a promoter which upon presence of the external stimulus is activated at least in or only in keratinocytes. Accordingly, the promoter can be a tissue-specific promoter, e.g. the KRT5 promoter for expression in keratinocytes.

A preferred additional expression cassette encoding meprin α for introduction into the genome of the rodent by genetic manipulation comprises the coding sequence of meprin α and a first promoter, which is inactive in the absence of an external stimulus, or which first promoter is arranged in combination with two recognition sites of a recombinase in a configuration in which the first promoter is inactive in respect of the meprin α coding sequence, and the animal further comprises a second expression cassette encoding the recombinase specific for the recognition sites under the control of an inducible promoter. In this embodiment, the second expression cassette forms the promoter inducible by an external stimulus, as the expression of the recombinase results in an activation of the expression cassette encoding meprin α. The two recognition sites can be arranged between the first promoter and the coding sequence for meprin α, and a stop element and optionally a selection marker can be arranged between the two recognition sites. Alternatively or additionally, the two recognition sites can be arranged to flank the first promoter or to flank the coding sequence of meprin α, so that the recombinase can convert the originally inactive arrangement of the promoter or of the coding sequence into an active arrangement, in which the first promoter drives expression of meprin α. The first promoter can be a strong constitutive promoter, e.g. the CAG promoter described by Miyazaki et al., Gene 79, 269-277 (1989). Generally, the recognition sites for a recombinase are arranged such that the recombinase can excise an element that otherwise prevents transcription of the meprin α encoding sequence, or the recognition sites can be arranged such that the recombinase turns a genetic element from an original inactive orientation into an active orientation that results in transcription of the meprin α encoding sequence.

In embodiments comprising a coding sequence for recombinase, the rodent preferably contains the ERT2 system which in the presence of tamoxifen, e.g. for Cre recombinase, results in translocation of recombinase from the cytosol into the nucleus.

Optionally, the rodent is genetically manipulated to overexpress meprin α in cells which express a peptidase, e.g. at least one of trypsin, plasmin, neutrophil elastase, and/or preferably at least one kallikrein, especially preferred in cells which express KLK5, and/or RgpB. Accordingly, the genetic manipulation can be directed to at least one cell type expressing a peptidase, e.g. by genetic manipulation of the rodent using e.g. viral vector particles which have preference for the at least one cell type. Alternatively, essentially all cells of the rodent may be genetically manipulated for overexpression of meprin α at presence of an external stimulus only.

Optionally additionally, nucleic acid constructs for genetic manipulation can comprise an expression cassette encoding at least one peptidase, e.g. trypsin, plasmin, neutrophil elastase, and/or preferably at least one kallikrein, KLK5, and/or RgpB.

The invention is now described by way of examples and with reference to the figures, which show in
- Fig. 1 A) to C) schematic drawings of nucleic acid constructs,
- Fig. 2 A) a schematic drawing of a nucleic acid construct of the invention,
- Fig. 2 B) a schematic drawing of the time course of a process of the invention,
- Fig. 2 C) a graph of body weights of mice of the invention and of control mice,
- Fig. 3 A) a Western blot for overexpression of meprin α from a nucleic acid construct of the invention in mice,
- Fig. 3 B) a graph of the enzymatic activity of meprin α in skin samples of mice of the invention,
- Fig. 3 C) electron microscopic pictures of skin cross-section of a control mouse and of a mouse according to the invention,
- Fig. 3 D) a graph of the thickness of the epidermis of control mice and of mice according to the invention,
- Fig. 4 A) results of blood cell counts,
- Fig. 4 B) photographs of the inside of skin samples from control mice and from mice of the invention,

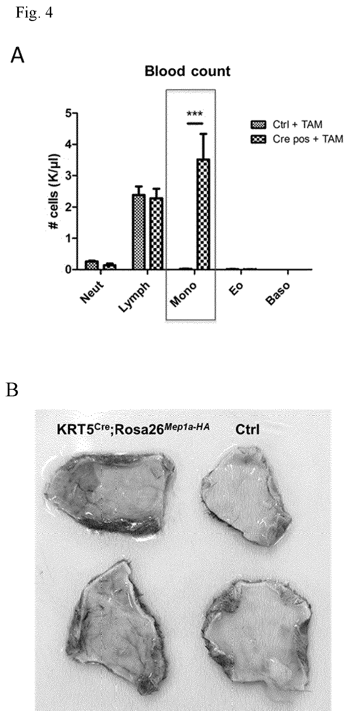

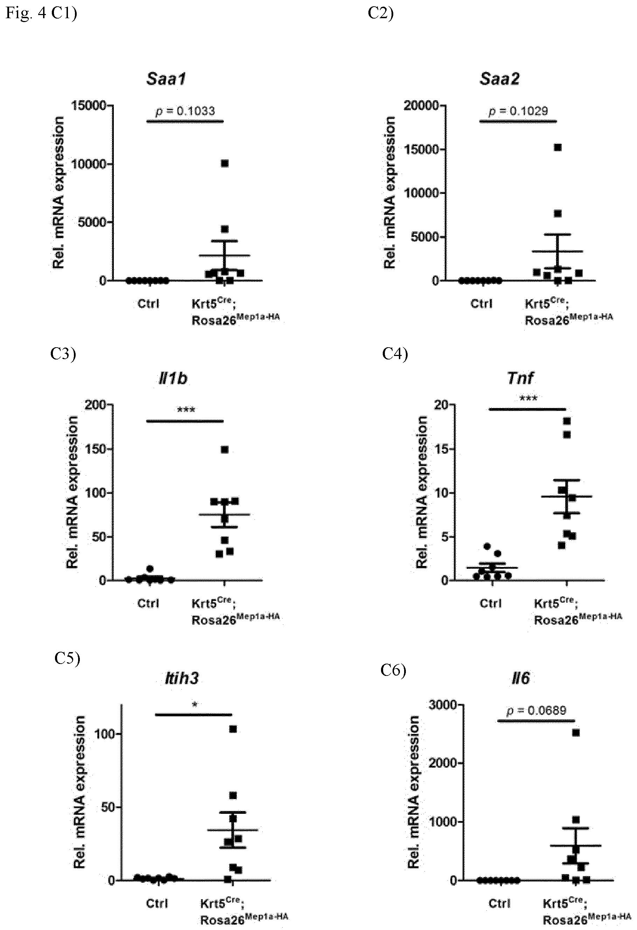

- Fig. 4 C1) to C6) show the results of RNA analyses of the acute-phase proteins and inflammatory cytokines,

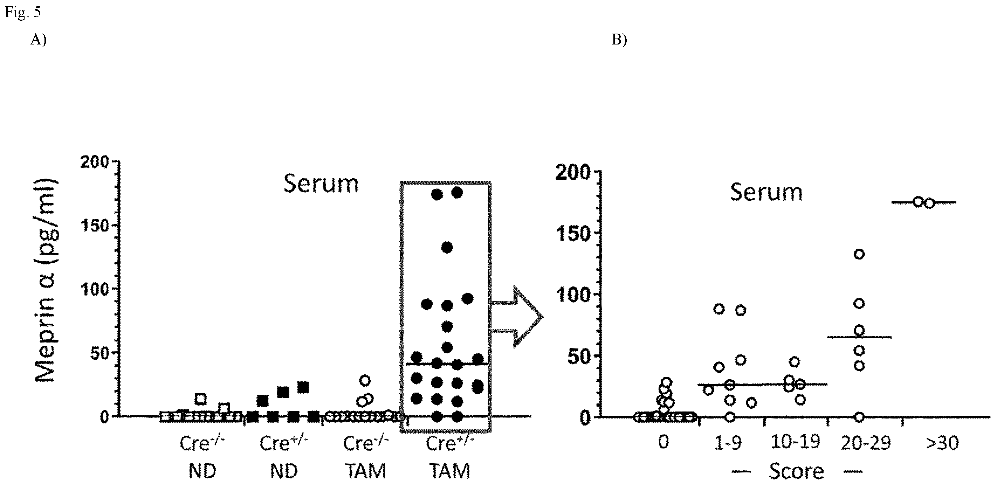

- Fig. 5 A) a graph of meprin α protein levels in serum of control mice and mice of the invention, and in B) disease scores of mice of the invention,

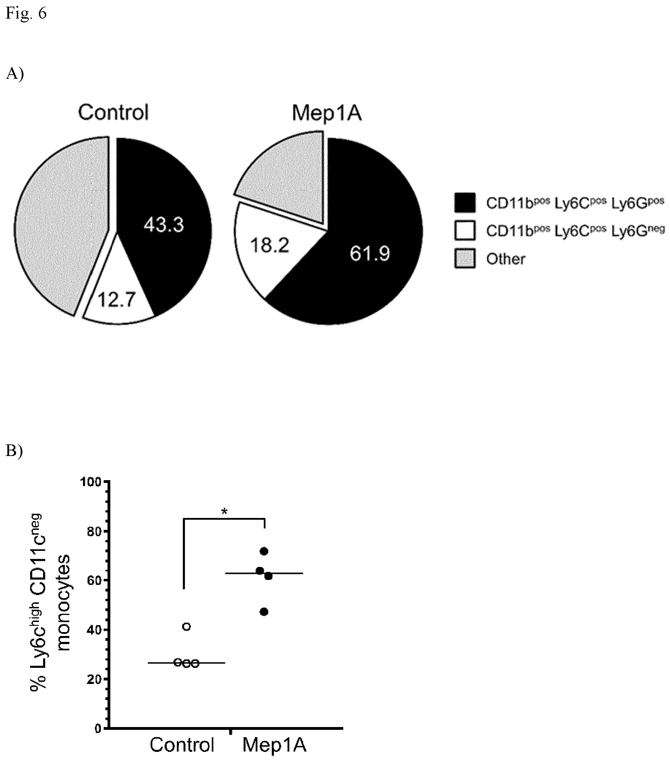

- Fig. 6 A) pie charts showing the percentages of mature and premature neutrophils in the bone marrow of control mice and of mice of the invention,
- Fig. 6 B) a graph of inflammatory monocytes in the blood of control mice and of mice of the invention, and in

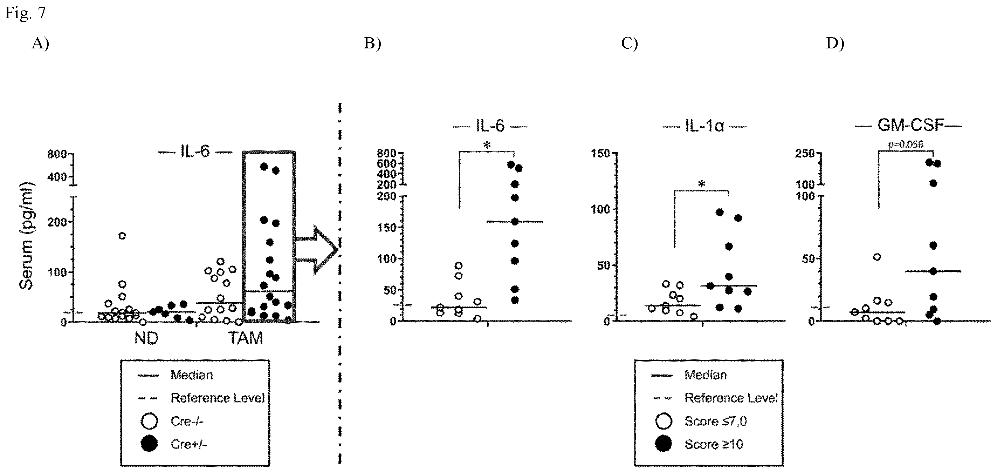

- Fig. 7 A) to D) graphs of serum levels of inflammatory cytokines IL-6, IL-1a and GM-CSF in control mice and mice of the invention.

### Example: Generation of genetically manipulated mouse that develops hyperkeratosis and sepsis following administration of an external stimulus

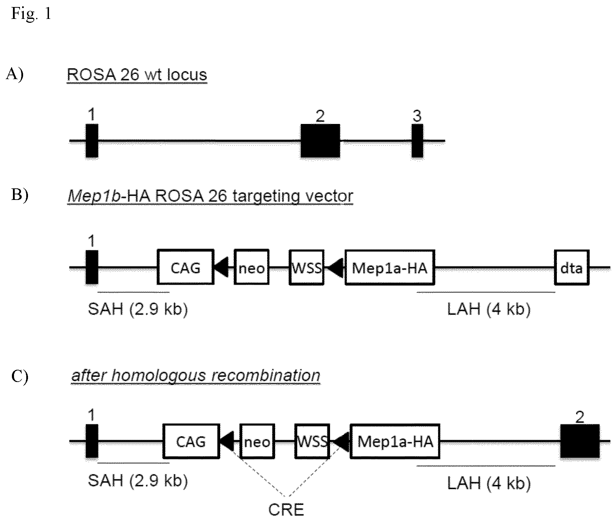

As an exemplary non-human mammal, mice (strain C57Bl/6N) were used for genetic manipulation to overexpress meprin α under the control of a promoter that is activated by an external stimulus. An expression cassette containing the cDNA of the murine meprin α coding sequence (Mep1a) with a CAG promoter and between these, flanked by recombinase recognition sites, a stop element and the neomycin resistance gene as a selection marker, was inserted into the Rosa26 locus. The wild type ROSA26 locus expresses two transcripts with no significant open reading frames (ORF) and a third in antisense orientation, which putatively encodes a protein, and which is not affected by the insertions. Targeting to the ROSA26 locus is conveniently achieved by introducing the desired gene into the first intron of the locus. The Rosa26 locus, wild-type, is schematically shown in Fig. 1A), comprising the sites exon 1 (reference numeral 1 in Fig. 1), exon 2 (reference numeral 2 in Fig. 1) and exon 3 (reference numeral 3 in Fig. 1).

Fig. 1B) shows the nucleic acid construct containing the expression cassette comprising the element short arm of homology (SAH), the CAG promoter, a first recombinase recognition site (◄), the neomycin resistance gene (neo), Westphal stop sequence (WSS) as a stop element, a second recombinase recognition site (◄, loxP), the coding sequence for meprin α with a C-terminal HA tag (MEP1a-HA), long arm of homology (LAH), and diphtheria toxin fragment A (dta), is schematically shown.

Fig. 1C) schematically shows the nucleic acid construct containing this expression cassette after insertion into the genomic DNA by homologous recombination in the Rosa26 locus, and indicates the activity of Cre recombinase (CRE) to excise the resistance gene (neo) and the stop element (WSS) that are flanked by the recombinase recognition sites.

The nucleic acid sequence of the construct shown in Fig. 1B) is given as SEQ ID NO: 1.

The coding sequence of meprin α (nucleotides No. 4573 to 6852 of SEQ ID NO: 1) corresponds to the cDNA sequence of the homologous meprin α, in this case the mouse sequence. The coding sequence for meprin α contained an additional tag-sequence encoding HA at its 3'-terminus (nucleotides No. 6853 to 6906 of SEQ ID NO: 1), resulting in the meprin α comprising the amino acid sequence of SEQ ID NO: 2 with an additional C-terminal HA tag. The HA tag is derived from the human influenza hemagglutinin and has the amino acid sequence YPYDVPDYA (SEQ ID NO: 3). Upon cleavage of the primary translation product of meprin α by a proprotein convertase on the natural secretory pathway, the C-terminus including the HA tag is cleaved off and therefore is assumed to not affect the activity of the resulting active meprin α. However, the C-terminal tag can be used to distinguish the translation product originating from the expression cassette from endogenous meprin α. The nucleic acid construct comprised regions of homology to the murine Rosa26 locus at both its ends for integration into this genomic locus. As a reporter gene, the nucleic acid construct contained the coding sequence for enhanced green fluorescent protein (EGFP). Between the CAG promoter (nucleotides 989 to 1576 of SEQ ID NO: 1) and the coding sequence for meprin α, there is the Westphal stop sequence (WSS, nucleotides 3217 to 4514 of SEQ ID NO: 1) and a neomycin resistance as a selection marker (nucleotides 2116 to 2919 of SEQ ID NO: 1) flanked by recombinase recognition sites (LoxP, nucleotides 2021 to 2054 and nucleotides 4527 to 4560 of SEQ ID NO: 1).

The mice which contained the inserted nucleic acid construct in their Rosa26 locus were mated with Krt5-Cre driver mice (strain B6N.129S6(Cg)-Krt5tm1.1(cre/ERT2)Blh/J ) that contain an expression cassette for recombinase Cre under the control of the keratin 5 promoter which is inducible by tamoxifen. In this embodiment, tamoxifen is the external stimulus, and the expression cassette for the recombinase forms the inducible promoter, as activity of the recombinase effects excision of the stop element, and hence the CAG promoter becomes active to translate meprin α. In addition, the mice contained the ERT2 system, which results in the translocation of cytosolic Cre into the nucleus upon presence of tamoxifen, which is preferable for time-dependent expression of meprin α in adult mice.

Recombinant mice that were identified to contain this nucleic acid construct were shown to exhibit a phenotype resembling sepsis and/or hyperkeratosis in 100% of the animals subsequent to administration of tamoxifen, but in the absence of tamoxifen these mice did not show any obvious phenotypic differences from the original strains.

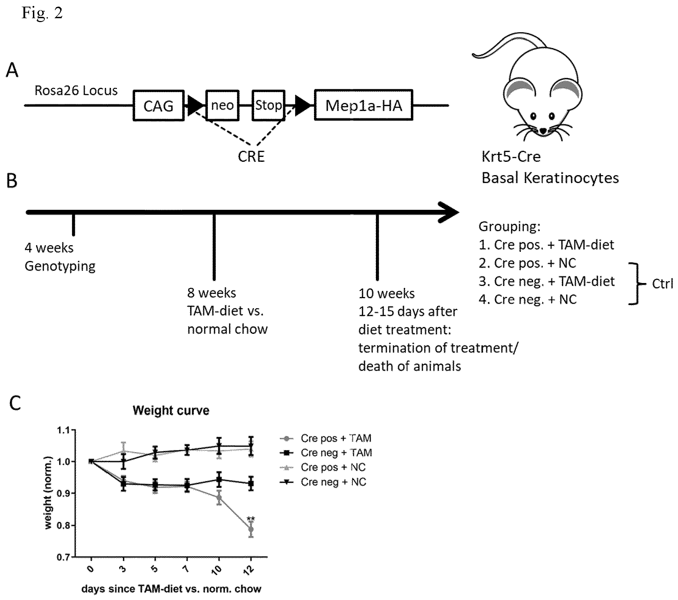

Fig. 2A) schematically shows a nucleic acid construct for overexpressing meprin α, which construct is integrated at the Rosa26 locus into the genome. Generally preferred, the recombinase recognition sites (►, loxP) flanking the stop element, which is excised by the recombinase (Cre) are arranged in the same orientation. The animal of the invention in addition to this nucleic acid construct for overexpressing meprin α contains an expression cassette for the cognate recombinase, in this embodiment under the control of a keratinocyte-specific promoter (Krt5-Cre).

A process for inducing sepsis and/or hyperkeratosis in the mice is shown in Fig. 2 B), with genotyping the mice at the age of 4 weeks, and at 8 weeks administering the external stimulus for overexpression of meprin α by feeding a tamoxifen-containing diet (TAM-diet), or normal feed without tamoxifen (normal chow, NC). For comparison, the genetically manipulated mice without being crossed with Krt5-Cre driver mice (Cre neg.) were treated with normal feed (NC) or with tamoxifen-containing diet (TAM-diet). None of the controls developed sepsis or hyperkeratosis. It was found that at 10 weeks, i.e. 12 to 15 days subsequent to the administration of tamoxifen, only the recombinant mice that are genetically manipulated for overexpression of meprin α according to the invention die from sepsis, or are euthanized after developing a state of sepsis.

Fig. 2C) shows the body weights determined for these animals (Cre pos + TAM: n = 7; Cre neg + TAM: n = 6; Cre pos + NC: n = 6; Cre neg + NC: n = 5 per group). It can be seen that presence of tamoxifen resulted in a weight loss, which gave a constant lower weight (10% weight loss compared to initial weight at week 8) also for the comparative mice which contained the nucleic acid construct comprising the expression cassette for meprin α but did not contain the inducible promoter formed by the expression cassette for recombinase Cre. Only the mice according to the invention suffered subsequent further weight loss (20% weight loss compared to the initial weight at week 8) at day 7 to 12, indicating the effects of sepsis. The control mice that were fed normal feed without tamoxifen as the external stimulus were not affected by the nucleic acid construct comprising the expression cassette for meprin α in combination with presence or absence of the expression cassette for recombinase Cre.

The expression of meprin α was tested in skin lysates of these mice by Western blotting, using an anti-HA antibody (C29F4 obtainable from Cell Signaling Technology) for detecting the HA-tag of meprin α originating from the nucleic acid construct comprising the expression cassette for meprin α. In short, skin was snap frozen in liquid nitrogen or directly pulverized in a nitrogen cooled mortar. Skin lysis buffer (50 mM Tris-HCl [pH 6.8], 150 mM NaCl, 1% [v/v] Triton X-100, 1% [w/v] SDS, 2 mM EDTA, Complete^{®} Protease Inhibitor Cocktail [Roche]) was added to the skin powder and incubated for 24 h at 4°C under rolling conditions. Skin lysates were centrifuged at 13.000 rpm, 4°C for 30 min and transferred into new microcentrifuge tubes. Total protein concentration was measured by BCA assay and 50 µg of denatured skin lysates used for SDS-PAGE and Western Blot analysis.

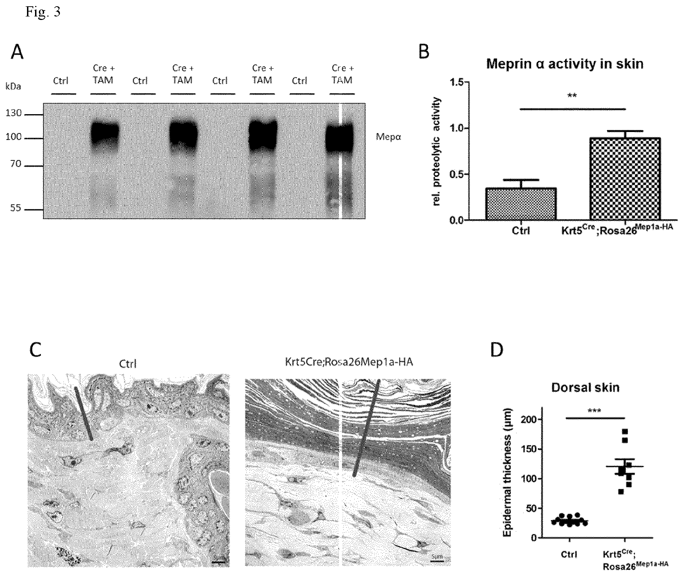

Fig. 3A) shows the Western blot and the band detected by the anti-HA antibody shows a molecular weight corresponding to that of meprin α. The activity of meprin α originating from the nucleic acid construct comprising the expression cassette for meprin α was determined by analysing the proteolytic activity in skin lysates. Fig. 2B) shows the increase of proteolytic activity in the skin samples of mice (Krt5^{Cre}, Ros26^{Mep1a-HA}) according to the invention in the presence of the external stimulus activating overexpression of meprin α, showing that the translation product of the nucleic acid construct comprising the expression cassette for meprin α generated enzymatically active meprin α.

Fig. 3C) shows pictures of transmission electron microscopy of skin samples of a comparative mouse (Ctrl) and of a mouse according to the invention (Krt5Cre;Rosa26Mep1a-HA). This shows a significant thickening (indicated by inserted line) of the epidermis and by enhanced terminal differentiation of keratinocytes in the mouse which according to the invention is genetically manipulated to overexpress meprin α in the presence of an external stimulus. Fig. 3D) shows measurement results for the epidermal thickness of dorsal skin samples of comparative mice (Ctrl) and of mice according to the invention (Krt5^{Cre}; Rosa26^{Mep1a-HA}), indicating the development of hyperkeratosis by the significantly increased thickness of the epidermis in the mice according to the invention.

Fig. 4A) shows the number of blood cells (x 1000 cells /µl (K/µl)) from analyses at day 12 of the feed containing tamoxifen of comparative mice that contained the expression cassette for meprin α but no expression cassette for Cre (Ctrl + TAM, left columns) and for mice according to the invention overexpressing meprin α (Cre pos + TAM, right columns), in both cases with tamoxifen containing feed. The results for neutrophils (Neut), lymphocytes (Lymph), eosinophils (Eo), and basophils (Baso) show no significant influence of the overexpression of meprin α, but the concentration of monocytes (Mono) is drastically increased to 1850 to 5540 cells per µl in the mice of the invention overexpressing meprin α compared to 10 to 30 cells per µl in comparative mice. This indicates a phenotype of sepsis in the mice that overexpress meprin α.

Fig. 4B) shows photographs of the inner sides of excised skin samples originating from mice according to the invention, which overexpress meprin α (Krt5^{Cre}; Rosa26^{Mep1a-HA}) and from comparative mice (Ctrl). These photographs show that in the mice overexpressing meprin α, the blood vessels in the sub cutis have stronger permeability than those of comparative mice. It is currently assumed that the stronger permeability of blood vessels in the sub cutis are induced by interactions of the endothelium with monocytic cells.

Fig. 4C) shows the results of RNA analyses of the acute-phase proteins and inflammatory cytokines. It was found that mRNA levels of Saa1 and of Saa2 in the mice according to the invention, which overexpress meprin α (Krt5^{Cre}; Rosa26^{Mep1a-HA}) were strongly increased, with individual values spreading by a factor of 10000 up to 15000 higher than in control mice that do not overexpress meprin α. The strong increase of levels of Saa1 and of Saa2 could indicate a more intense state of sepsis. Also for IL-6 (Il6), a much higher concentration was found in mice overexpressing meprin α, with the highest increase by a factor of 2500 compared to control mice. Also, genes for pro-inflammatory cytokines IL-1b (Il1b) and tumor necrosis factor (Tnf) as well as for acute phase protein Iith3 (Itih3) were significantly upregulated in mice overexpressing meprin α.

The log2 differences of protein concentrations were determined by mass spectrometry analyses for skin lysates from mice overexpressing meprin α to the protein concentrations for skin lysates from mice that were not manipulated to overexpress meprin α.

From alterations in the total proteins in mice overexpressing meprin α in comparison to mice without genetic manipulation to overexpress meprin α the acute-phase proteins alpha-1-acid glycoprotein 2, serum amyloid A-1, and serum amyloid A-2 were identified as 3 of the 7 most highly upregulated proteins. Other proteins that were found to be upregulated in the mice according to the invention were connected to skin damage and to proteolysis, like protein S100-A9, neutrophilic granule protein, and matrix metalloprotease-9. Decreased expression was found for suprabasin and metallothionein-4.

These results also indicate that the genetically manipulated animals of the invention which overexpress meprin α in the presence of an external stimulus generate a phenotype indicating sepsis.

The analytical results of monocytosis, of induction of pro-inflammatory cytokine expression, the weight loss and the sudden deaths of animals according to the invention indicated that the overexpression of meprin α in the animals according to the invention results in the development of acute inflammation responses, indicating sepsis.

Fig. 5 A shows a graph of serum levels of meprin α for control (comparative) mice carrying the Rosa26Mep1a-HA gene but no Krt5CreERT2 (Cre-/-), and for mice of the invention having both Rosa26Mep1a-HA and Krt5CreERT2 (Cre+/-). The mice had been fed either a tamoxifen-containing diet (TAM) or a normal diet without tamoxifen (ND) for 12 days. The expression of meprin α from the expression cassette that was introduced was analyzed in the serum of these mice by ELISA, detecting total murine meprin α.

Serum levels of meprin α are increased in Cre+/- TAM mice, which had a median serum meprin α concentration of 40 pg/mL (horizontal line). Notably, neither the presence of the Krt5CreERT2 gene alone (Cre+/- ND) nor the tamoxifen-containing diet alone (Cre-/- TAM) produced an increase in serum meprin α levels compared to control mice on a diet without tamoxifen (Cre-/- ND). Therefore, the mice of the invention provide a robust model for systemically inducible meprin α.

Fig. 5 B shows the correlation between serum levels of meprin α and a disease score ("Score") which is based on different factors, including body weight, behavioural abnormalities, as well as skin irritations and wounding. In particular, 0 disease score points correspond to no physical stress; 1-9 points correspond to minor physical stress; 10-19 points equal physical stress that requires medical treatment after 72 hours; >20 points correspond to severe physical stress that requires euthanasia of the animals. The results show that the serum concentration of meprin α positively correlates with disease score, wherein higher disease scores have higher median levels (horizontal bars) of meprin α, and those mice having the highest detected levels of meprin α showed the highest disease score. Further, only those mice of the invention that were fed a tamoxifen containing diet (Cre+/- TAM) showed a disease score above 0.

Fig. 6A) shows the proportions of mature and premature neutrophils in the bone marrow of meprin α overexpressing Krt5CreERT2;Rosa26Mep1a-HA mice of the invention (Mep1A), and of comparative mice having the Krt5CreERT2 gene alone (Control). Both the number of mature neutrophils having the cell surface markers (CD11bpos Ly6Cpos Ly6Gpos) and the number of premature neutrophils having the cell surface markers CD11bpos Ly6Cpos Ly6Gneg increased by a factor of ca. 1.4 in those mice overexpressing meprin α (Mep1A) compared to the control group without meprin α overexpression. Numbers refer to the percentages of those neutrophils as a proportion of all immune cells detected in bone marrow. These results indicate that those mice expressing meprin α also show increasing neutrophilia due to activated granulopoiesis.

Fig. 6B) shows the proportions of Ly6c^{high} CD11c^{neg} inflammatory monocytes in the bone marrow of Krt5CreERT2;Rosa26Mep1a-HA mice overexpressing meprin α (Mep1A), and of comparative mice (Control). The median proportion of inflammatory monocytes accounted for 63 % of all monocytes in meprin α overexpressing mice (Mep1A) and for 27 % of all monocytes in control mice (n = 4).

Data for Figs. 6A) and 6B) was obtained by flow cytometric identification of different immune cell populations. Cell surface markers CD3, CD4, CD8a, CD11b, CD19, CD64, I A/I-E, Ly6c and Ly6g were detected using marker-specific antibodies. Cells were separated and classified by size and granularity using forward and side scatter characteristics, and by the expression of the above cell surface markers by fluorescence detection.

Fig. 7 A) shows serum concentrations of inflammatory cytokine IL-6 in Rosa26Mep1a-HA control mice (Cre-/-, ∘), and in Krt5CreERT2;Rosa26Mep1a-HA mice of the invention (Cre+/-, •). Mice were either fed on a normal diet without tamoxifen (ND) or on a tamoxifen containing diet (TAM). The reference level shown on the Y axis marks the median value of Cre-/- ND mice. Median levels of serum IL-6 concentration were slightly increased in Cre-/- mice on TAM diet, and even further increased in Cre+/- mice on TAM diet. Serum IL-6 levels over 400 pg/mL were only observed in Cre+/- TAM mice, which overexpress meprin α.

Fig. 7 B), C), and D) show serum levels of inflammatory cytokines IL-6, IL-1α and GM-CSF in Cre+/- TAM mice. Mice were separated into groups depending on the observed disease score (score), which was determined as explained above. Those mice having a maximum score of 7 showed no median increase in serum IL-6 concentration, a slight increase in IL-1α and no increase in GM-CSF, all compared to the reference level. In contrast, those mice having a score of at least 10 showed a median increase of serum IL-6 levels by a factor of ca. 6.5, an increase in IL-1α by a factor of ca. 2.5 and an increase in GM-CSF by a factor of ca. 5, all compared to the corresponding levels of Score ≤ 7,0 mice. These results show that the disease score correlates with serum levels of inflammatory cytokines, further validating the mouse model of the invention.

Results shown in Fig. 7 were obtained using the LEGENDplex^{™} Mouse Inflammation Panel assay (BioLegend) for simultaneous quantification of mouse cytokines. The assay was used according to the manufacturer's instructions.

## Claims

1. Rodent that is genetically manipulated to develop the phenotype of sepsis and hyperkeratosis upon activation by an external stimulus, which rodent is genetically manipulated to contain an additional expression cassette encoding meprin α under the control of a promoter which upon presence of the external stimulus is activated at least or only in keratinocytes to express meprin α.

2. Rodent according to claim 1, **characterized in that** the expression cassette encoding meprin α contains a promoter that is cell-type specific for keratinocytes.

3. Rodent according to one of the preceding claims, **characterized in that** the coding sequence for meprin α is separated from its promoter by an intermediate stop element which is flanked by recognition sites for a recombinase and **in that** the mammal contains an expression cassette for the cognate recombinase under the control of a promoter which is induced by the presence of the external stimulus.

4. Rodent according to one of the preceding claims, **characterized in that** it is in addition genetically manipulated to contain an expression cassette encoding a peptidase.

5. Rodent according to one of the preceding claims, **characterized by** the phenotype being without administration of toxins or other pro-inflammatory stimuli and without mechanically destroying epithelia or tissues of the rodent.

6. Process for testing chemical compounds, comprising providing a rodent according to one of the preceding claims in which meprin α is expressed in keratinocytes, administering the external stimulus to the rodent for generating a phenotype of sepsis and of hyperkeratosis, and administering at least one chemical compound to the rodent.

7. Process according to claim 6, **characterized in that** no additional toxin or proinflammatory compound is administered to the rodent and no mechanical damage is inflicted upon the rodent.

8. Process for generating a rodent which develops a phenotype of sepsis and of hyperkeratosis , **characterized by** introducing into the rodent a genetic manipulation that results in the presence of a promoter which is inducible by presence of an external stimulus, which promoter upon presence of the external stimulus is activated at least or only in keratinocytes and which stimulus induces overexpression of meprin α.

9. Process for generating a phenotype of sepsis and/or of hyperkeratosis and/or of systemic inflammation in a rodent, comprising the steps of providing a rodent according to one of claims 1 to 5, and administering the external stimulus to the rodent.

10. Process according to claim 9, **characterized in that** it is without administration of any additional toxin or other pro-inflammatory stimuli, and without mechanically destroying epithelia or tissues of the rodent.

## Patentansprüche

1. Nagetier, das genetisch so manipuliert ist, dass es bei Aktivierung durch einen externen Stimulus den Phänotyp von Sepsis und Hyperkeratose entwickelt, wobei das Nagetier genetisch so manipuliert ist, dass es eine zusätzliche Expressionskassette enthält, die für Meprin α unter der Kontrolle eines Promotors kodiert, der bei Anwesenheit des externen Stimulus zumindest oder nur in Keratinozyten zur Expression von Meprin α aktiviert wird.

2. Nagetier nach Anspruch 1, **dadurch gekennzeichnet, dass** die Expressionskassette, die für Meprin a kodiert, einen Promotor enthält, der zelltypspezifisch für Keratinozyten ist.

3. Nagetier nach einem der vorhergehenden Ansprüche, **dadurch gekennzeichnet, dass** die kodierende Sequenz für Meprin α von ihrem Promotor durch ein intermediäres Stopp-Element getrennt ist, das von Erkennungsstellen für eine Rekombinase flankiert ist, und dass das Säugetier eine Expressionskassette für die passende Rekombinase unter der Kontrolle eines Promotors enthält, der durch die Anwesenheit des externen Stimulus induziert wird.

4. Nagetier nach einem der vorhergehenden Ansprüche, **dadurch gekennzeichnet, dass** es zusätzlich genetisch so manipuliert ist, dass es eine Expressionskassette enthält, die für eine Peptidase kodiert.

5. Nagetier nach einem der vorhergehenden Ansprüche, **dadurch gekennzeichnet, dass** der Phänotyp ohne Verabreichung von Toxinen oder anderen proinflammatorischen Stimulanzien und ohne mechanische Zerstörung von Epithelien oder Geweben des Nagetiers auftritt.

6. Verfahren zum Testen chemischer Verbindungen, das das Bereitstellen eines Nagetiers nach einem der vorhergehenden Ansprüche umfasst, in dem Meprin α in Keratinozyten exprimiert wird, das Verabreichen des externen Stimulus an das Nagetier, um einen Phänotyp der Sepsis und der Hyperkeratose zu erzeugen, und das Verabreichen mindestens einer chemischen Verbindung an das Nagetier.

7. Verfahren nach Anspruch 6, **dadurch gekennzeichnet, dass** dem Nagetier kein zusätzliches Toxin oder proinflammatorische Verbindung verabreicht wird und dem Nagetier kein mechanischer Schaden zugefügt wird.

8. Verfahren zur Erzeugung eines Nagetiers, das einen Sepsis- und Hyperkeratose-Phänotyp entwickelt, **dadurch gekennzeichnet, dass** in das Nagetier eine genetische Manipulation eingeführt wird die zum Vorliegen eines Promotors führt, der durch das Vorliegen eines äußeren Stimulus induzierbar ist, wobei der Promotor bei Vorliegen des äußeren Stimulus zumindest oder nur in Keratinozyten aktiviert wird und der Stimulus eine Überexpression von Meprin α induziert.

9. Verfahren zur Erzeugung Phänotyps der eines Sepsis und/oder Hyperkeratose und/oder der systemischen Entzündung in einem Nagetier, das die Schritte des Bereitstellens eines Nagetiers nach einem der Ansprüche 1 bis 5 und des Verabreichens des externen Reizes an das Nagetier umfasst.

10. Verfahren nach Anspruch 9, **dadurch gekennzeichnet, dass** es ohne Verabreichung eines zusätzlichen Toxins oder anderer proinflammatorischer Stimulanzien und ohne mechanische Zerstörung von Epithelien oder Geweben des Nagetiers erfolgt.

## Revendications

1. Rongeur génétiquement modifié pour développer le phénotype de la septicémie et de l'hyperkératose lors de l'activation par un stimulus externe, lequel rongeur est génétiquement modifié pour contenir une cassette d'expression supplémentaire codant pour la méprine α sous le contrôle d'un promoteur qui, en présence du stimulus externe, est activé au moins ou uniquement dans les kératinocytes pour exprimer la méprine a.

2. Rongeur selon la revendication 1, **caractérisé en ce que** la cassette d'expression codant pour la méprine α contient un promoteur spécifique du type cellulaire des kératinocytes.

3. Rongeur selon l'une des revendications précédentes, **caractérisé en ce que** la séquence codante de la méprine α est séparée de son promoteur par un élément d'arrêt intermédiaire flanqué de sites de reconnaissance pour une recombinase, et **en ce que** le mammifère contient une cassette d'expression pour la recombinase correspondante sous le contrôle d'un promoteur induit par la présence du stimulus externe.

4. Rongeur selon l'une des revendications précédentes, **caractérisé en ce qu'**il est en outre génétiquement modifié pour contenir une cassette d'expression codant pour une peptidase.

5. Rongeur selon l'une des revendications précédentes, **caractérisé en ce que** le phénotype est obtenu sans administration de toxines ou d'autres stimuli pro-inflammatoires et sans destruction mécanique de l'épithélium ou des tissus du rongeur.

6. Procédé de test de composés chimiques, comprenant la fourniture d'un rongeur selon l'une des revendications précédentes, dans lequel la meprine α est exprimée dans les kératinocytes, l'administration du stimulus externe au rongeur pour générer un phénotype de septicémie et d'hyperkératose, et l'administration d'au moins un composé chimique au rongeur.

7. Procédé selon la revendication 6, **caractérisé en ce qu'**aucune toxine ou aucun composé pro-inflammatoire supplémentaire n'est administré au rongeur et qu'aucun dommage mécanique n'est infligé au rongeur.

8. Procédé pour générer un rongeur qui développe un phénotype de septicémie et d'hyperkératose, **caractérisé par** l'introduction dans le rongeur d'une manipulation génétique qui entraîne la présence d'un promoteur inductible par la présence d'un stimulus externe, lequel promoteur, en présence du stimulus externe, est activé au moins ou uniquement dans les kératinocytes et lequel stimulus induit une surexpression de la méprine a.

9. Procédé pour induire un phénotype de septicémie et/ou d'hyperkératose et/ou d'inflammation systémique chez un rongeur, comprenant les étapes consistant à fournir un rongeur selon l'une des revendications 1 à 5, et à administrer le stimulus externe au rongeur.

10. Procédé selon la revendication 9, **caractérisé en ce qu'**il se déroule sans administration d'aucune toxine supplémentaire ni d'autres stimuli pro-inflammatoires, et sans destruction mécanique des épithéliums ou des tissus du rongeur.
